Europäisches Patentamt

European Patent Office

Office européen des brevets

(19)

(11) **EP 1 526 177 A1**

(12) **EUROPEAN PATENT APPLICATION**

(43) Date of publication:
**27.04.2005 Bulletin 2005/17**

(51) Int Cl.$^7$: **C12N 15/11**

(21) Application number: **03292666.9**

(22) Date of filing: **24.10.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(71) Applicants:
- **Institut Curie
  75248 Paris Cedex 05 (FR)**
- **CENTRE NATIONAL DE LA RECHERCHE
  SCIENTIFIQUE (CNRS)
  75794 Paris (FR)**
- **MUSEUM NATIONAL D'HISTOIRE NATURELLE
  75231 Paris Cedex 05 (FR)**

- **INSTITUT NATIONAL DE LA SANTE ET DE LA
  RECHERCHE MEDICALE (INSERM)
  75654 Paris Cédex 13 (FR)**

(72) Inventors:
- **Dutreix, Marie
  94240 L'Haye les Roses (FR)**
- **Sun, Jian-Sheng
  93600 Aulnay sous Bois (FR)**

(74) Representative: **Peaucelle, Chantal et al
  Cabinet Armengaud Ainé
  3, avenue Bugeaud
  75116 Paris (FR)**

(54) **Nucleic acids useful for triggering tumor cell lethality**

(57)     The invention relate to double stranded nucleic acid fragments comprising a chemically modified backbone and at least 4-1000 bp, preferably 8-500 bp, and most preferably 12-100 bp.

Application as adjuvant compositions to be used in association with a DNA breaking treatment, particularly radiotherapy or chemotherapy, in combination with a pharmaceutically acceptable carrier, in an efficient amount to be introduced in the tumoral cell nuclei in order to trigger DNA repair induced lethality (DRIL in short) of tumoral cells / tissues.

**EP 1 526 177 A1**

## EP 1 526 177 A1

**Description**

The invention relates to nucleic acids useful as tools for triggering cell lethality of tumors submitted to anticancer therapies.

Chemotherapy, radiotherapy, alone or combined together with surgery, are essential therapeutic tools against human cancer.

Ionizing radiation causes direct DNA breaks and triggers cell / tissue death. In the past years, many studies have focused on biological mechanisms related to the ionizing radiation response, in order to gain insights into the complexity of phenomena underlying radio-sensitivy or radio-resistance of tumor cells. The understanding of the different pathways which finely regulate the response to ionizing radiation is an important step to identify molecular targets for new drugs and genetic therapies that, in association with radiotherapy, could improve the chance of recovery from tumors highly resistant to radiation, as brain or head and neck tumors.

DNA breaks or damages are also observed when submitting tumors to chemotherapy treatments. The use of chemotherapeutic agents such as inhibitors of topoisomerases, inhibitors of the mitotic spindle, such as Taxol or Nocodasol, damaging agents, such as 5-fluoro-uracil or cisplatin, will indirectly induce said DNA breaks and damages.

Among the genes of particular interest to be targeted in combination with ionizing radiation are those involved in the regulation of radiation-induced lethality mechanisms such as necrosis or apoptosis. Cell death induced by ionizing radiation and chemotherapy treatments mostly depends on defects in double-strand DNA breaks (DSB in short) repair.

Two mechanisms are involved in the repair of these lesions: non homologous end-joining (NHEJ) involving proteins such as Ku70 and 80 which recognize and bind DSB ends and homologous recombination (HR) (reviewed by Jackson, 2002). Targeting genes involved in these two main DSB repair pathways leads to little or moderate radiosensitivity depending on the used approaches and cancer cell lines (Belenkov et al., 2002; Marangoni et al. 2000; Ohnishi et al., 1998). As the NHEJ and the HR pathways seem to be both involved in the DSB repair in a complementary manner, according to cell cycle phases, one of the possible explanation of the moderate effect obtained even when strong inhibition of protein expression is achieved, could reside in a residual repair activity which would partially compensate the repair defect obtained by targeting one specific repair pathway.

A recent work reports moderate results obtained in mice bearing human tumor xenograft treated with above described Ku 70 antisense (Li et al, 2003).

Up to now, nothing was proposed to overcome the drawbacks of the resistance acquired by repeated radiotherapeutic or chemotherapeutic treatments.

The inventors have found that the tumor sensitivity to direct or indirect DNA damaging anticancer therapies can be enhanced by using chemically modified double stranded nucleic acid molecules, acting as mimetics of broken DNA fragments and recognized as DSB sites induced by the DNA damaging treatments, but having a non replicative structure due said modifications.

An object of the invention is then to provide such double stranded nucleic acid fragments, also designated DNA repair induced lethality (DRIL in short) molecules in the following.

Another object of the invention is to provide a new molecular strategy to target all repair processes of double strand breaks in human cancer cell/ tissue body, especially with a radio-and/ or chemo-resistant phenotype.

More particularly, the invention aims at providing new DRIL molecules to be used in combination with DNA breaking agent(s) and a method for treating cancer combining the use of said DRIL molecules with a DNA damaging anticancer therapies.

Another object of the invention relates to the use of DRIL molecules for making anti-tumoral drugs for treating tumors, particularly highly resistant tumors to radio-and/or chemotherapies.

The double stranded nucleic acid fragments of the invention comprise a chemically modified backbone of at least 4-1000 bp, preferably 8-500 bp and most preferably 12-100 bp.

Said modifications are such that:

- the double stranded DRIL molecules are capable of being uptaken by cell / tissue body into the cell nucleus;
- the free ends of the DRIL molecules are recognizable by the DNA binding proteins involved in double strand breaks repair and damaging signalling, whereby the DRIL molecules is amenable by said enzymes to be incorporated in the tumoral cell genomic DNA;
- the double stranded DRIL molecules are stable and resistant to nucleases.

The double-stranded fragments according to the invention have a phosphodiester backbone or may comprise other backbone chemical groups or mixtures of chemical groups.

It may also have sugar mimetics such as cyclobutyls or other carbocyclics or hexitol in place of the pentofuranosyl group.

Preferred fragments comprise one or several chemical groups at the end of each strand. Preferred chemical

2

groups comprise phosphorothioates.

Other modified backbones of the invention comprise methylphosphonates, phosphoramidates, morpholino nucleic acid, 2'-O,4'-C methylene/ethylene bridged locked nucleic acid, peptide nucleic acid (PNA), and short chain alkyl, or cycloalkyl intersugar linkages or short chain heteroatomic or heterocyclic intrasugar linkages of variable length, or any modified nucleotides known by skilled person.

US patent No. 5,677,437 describes heteroaromatic oligonucleoside linkages. Nitrogen linkers or groups containing nitrogen can also be used to prepare oligonucleotide mimics (U.S. Patents No. 5,792,844 and No. 5, 783,682). U.S. Patent No. 5,637,684 describes phosphoramidate and phosphorothioamidate oligomeric compounds. Also envisioned are oligonucleotides having morpholino backbone structures (U.S. Patent No. 5,034,506). In other embodiments, such as the peptide-nucleic acid (PNA) backbone, the phophodiester backbone of the oligonucleotide may be replaced with a polyamide backbone, the bases being bound directly or indirectly to the aza nitrogen atoms of the polyamide backbone. Other synthetic oligonucleotides may contain substituted sugar moieties comprising one of the following at the 2' position: OH, SH, $OCH_3$, $SCH_3$, F, OCN, $OCH_2CH_2OCH_3$, $O(CH_2)nNH2$ or $O(CH_2)_nCH3$ where n is from 1 to about 10 ; C1 to C10 lower alkyl, substituted lower alkyl, alkaryl or aralkyl ; Cl ; Br ; CN ; $CF_3$ ; $OCF_3$ ; O- ; S- ; or N-alkyl ; O-, S-, or N-alkenyl ; $SOCH_3$ ; $SO_2$ $ONO_2$ ; $NO_2$ $N_3$ ; $NH_2$ ; heterocycloalkyl ; heterocycloalkaryl ; aminoalkylamino ; polyalkylyamino ; substitued silyl ; or a group for improving the pharmacodynamic properties of an oligonucleotide, and other substituents having similar properties.

The DRIL molecules are essentially based on natural nucleotides either 2'-deoxynucleotides or 2'-ribonucleotides and optionally comprise one or several modified nucleotides and/or nucleobases other than adenine, cytosine, guanine, thymine and uracil.

Appropriate nucleobases other than the usual bases are for example C5-methylcytosine, pseudoisocytosine, C5-propynyluracil, N7-deazaguanine, N7-glycosylated guanine, or alpha anomer.

The chemically modified DRIL molecules, which will be in the cell in the tissue or in the body when they are irradiated or treated by chemotherapies, will be either incorporated into genomic DNA at the DSB sites, or recognized as DSB sites induced by ionizing radiation by cellular DNA repair mechanism as NHEJ or HR. Then, they will be bound by DSB repair proteins, either being integrated into the broken chromosomes or saturating the repair system.

According to an embodiment of the invention, said DRIL molecules further comprise at least one embedded element which hampers DNA replication or repair process.

Said non-replicable element(s) can be incorporated at the internal position or at the end of the double-stranded fragment. It (they) may comprise:

a) a unit which cannot be used as a template for DNA replication, such as a polyethyleneglycol chain, preferably a hexaethyleneglycol chain, or any hydrocarbon chain, eventually interrupted and/ or substituted by one or more heteroatoms e.g. oxygen, sulfur, nitrogen, or heterogroups comprising one or more of these heteroatoms;
b) a unit which is a blocking element as it is not amenable by DNA polymerases or exonucleases, such as any 3'-modified nucleotides, or other ones known by skilled person;
c) a native oligonucleotide, such as $T_n$, when used in the loop of an hairpin fragment.

The DRIL molecules of the invention can be formed by hybridizing two substantially complementary strands or by folding of a single strand (hairpin formation), or by any method of DNA amplification followed by chemical modification.

Said strands are made by chemical synthesis or by molecular biology.

The experiments carried out in cell culture and xenografted tumors on nude mice have shown that said DRIL molecules trigger cell/tissue lethality of tumors submitted to a radio-and/ or chemotherapy.

The invention thus also relates to adjuvant compositions to be used in association with a DNA breaking treatment, said compositions comprising a DRIL molecule such as above defined, in combination with a pharmaceutically acceptable carrier, in an efficient amount to be introduced in the tumoral cell genome.

The invention also relates to a method for promoting tumor sensibility to anticancer therapies which comprises, in association,

- introducing into tumor cell/tissue a nucleic acid fragment such as above defined, and
- inducing in cells, DNA breakage by a DNA damaging method.

Advantageously, said method comprises coupling the treatment with DRIL molecules with a double chemotherapy. For example 5-FU and CPT 11 are injected together 3 times, 3 consecutive days, spaced by a full week of rest. Alternatively the treatment with DRIL molecules is coupled with radiotherapy. The protocol may comprise administration of DRIL molecules several hours before irradiation, for example 5 hours, and 3 times a week, a total dose of irradiation corresponding to 30 Gy over 6 weeks of treatments. The use of a fractionated irradiation is particularly efficient.

In a preferred embodiment, the DRIL molecules are chemically modified DRIL molecules such as above defined.

In another embodiment, the DRIL molecules are not chemically modified and correspond to *native NA fragments,* but exhibit the characteristics of chemically modified fragments, particularly have the number of base pairs and properties defined with respect to said chemically modified DRIL molecules.

More particularly DNA strand breakage is achieved by ionized radiation (radiotherapy) or chemical reaction (chemotherapy).

Such a method is a new therapeutic adjuvant in conjunction with DNA damaging therapies to tumors.

The invention also relates to the use of said non-chemically modified DRIL molecules for making anti-tumoral drugs for treating tumors, particularly highly resistant tumors to radio-and/or chemotherapies, said drugs being used in association with a DNA breaking treatment, particularly radiotherapy or chemotherapy.

In vivo, the chemically modified or non-modified DRIL molecules are administered by any appropriate route such as oral, or intravenous, or intratumoral administration, or sub-cutaneous injections, or others.

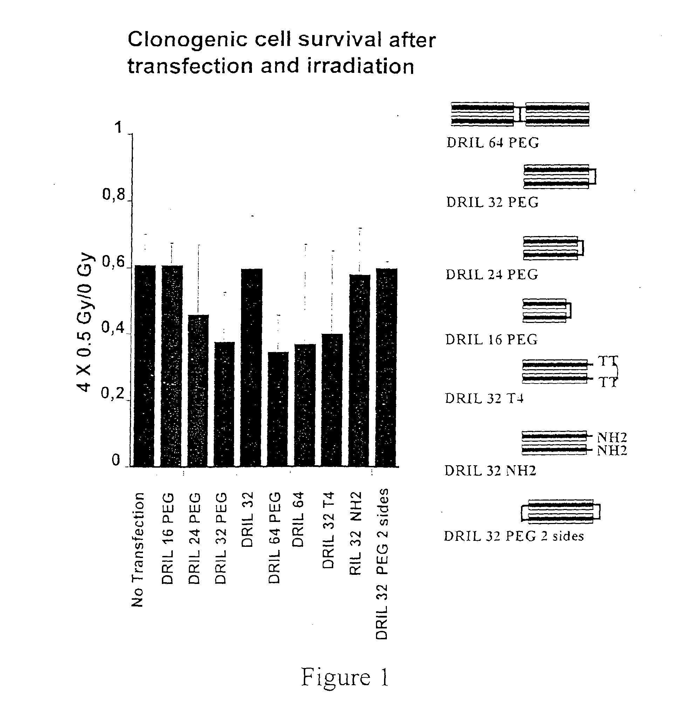

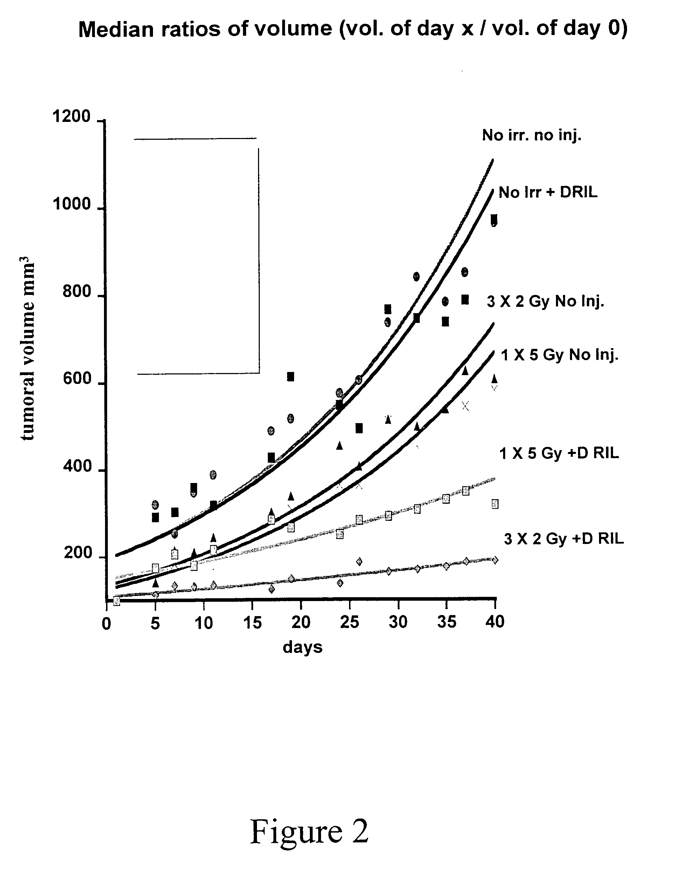

Others characteristics and advantages of the invention will be given in the following examples, with reference to figures 1 and 2:

- Figure 1 illustrates DRIL molecules used according to the invention and gives the clonogenical survival after transfection and irradiation of different DRIL molecules at 83 nM corresponding to 1 μg of DNA for $2 \times 10^5$ cells; and
- Figure 2 gives the relative tumor growth as the median of the Vt:Vo ratio (Vt = median tumor volume on a given day during the treatment and Vo is the mean tumor volume at the beginning of the treatment).

Examples

Example 1: Design of DRIL molecules according to the invention

A 32-bp double helix was taken as a basic unit of DRIL molecules in the experiments disclosed hereinafter.

The following sequence (abbreviated as 32) was chosen

5'-ACGCACGGGTGTTGGGTCGTTTGTTCGGATCT-3'

3'-TGCGTGCCCACAACCCAGCAAACAAGCCTAGA-5'

Linear and hairpin DNA fragments were designed and are schematized on figure 1 where the rectangle = 32-bp DNA fragment and vertical black bar =PEG

Example 2: Assay in cell culture

The DRIL molecules have been tested *in vitro* in combination with fractionated gamma irradiation, using a radio-resistant human cancer cell line derived from a female cervix carcinoma (HeLa).

Transfection of HeLa cells was achieved using a new class of activated-dendrimer reagent (SuperFect) which has been currently used in the past few years for its low cytotoxicity and high transfection efficiency in mammalian cell lines. 24 hours after seeding plating, cells were exposed to SuperFect-DRIL complexes during 8 hours and irradiated with 4 fraction of 0.5 Gy given each two hours. Then cells are washed and left in normal medium for 16 hours before seeding for clonogenical survival assay.

The fractionated irradiation has been privileged insofar as the totality of clinical protocols involving radiotherapy relay on fractionation of the total dose to minimize toxic effects of ionizing radiation. γ-irradiation was delivered by a $^{137}$CS source at a dose rate of 0.97 Gy/mn.

In figure 1 the radiation sensitivity is represented as survival fraction at 4 x 0.5 Gy plotted for various DRIL molecules transfected at 83 nM (corresponding to 1 μg of DNA for $2.10^5$ cells plated 24 hours before). The DRIL molecules used differ for size (from 16 to 64 bp) and chemical structure modification: polyethylenglycol chain (PEG) at one ore two extremities of the molecules, or inside (DRIL 64 PEG), amines or $T_4$ loops.

The percent of survival of not transfected HeLa cells is about 60%. Some DRIL molecules, like DRIL 16 or DRIL 32 carrying PEG at the two sides, did not sensitize HeLa cells. By contrast, cells transfected with DRIL 32 and 64 PEG showed a significant increase in radiation sensitivity (between 30% and 40% of survival after irradiation).

Example 3: Assay in animal model

*In vivo* studies were carried out on female athymic nude mice bearing human tumor xenograft obtained by subcutaneous injection of HEP2 cells in the right flank.

The tumor size was measured twice a week and tumor volume was estimated from two dimensional tumor measurements by the formulae:

$$\text{tumor volume} = \text{length (mm)} \times \text{width}^2 \ (\text{mm}^2)/2$$

The DRIL 32 PEG, associated with the transfectant reagent Superfect*, has been tested. Mice were injected with 20 µg of DRIL mixed with a ratio 1/5 of Superfect*. Two protocols of irradiation were compared: two fractionated schema with 3 x 2 Gy or 5 x 1 Gy per week delivered locally using a conformational system. The total dose delivered was 30 Gy over six weeks of treatment. The intra-tumoral injection is made 5 hours before irradiation and 3 times per week.

Six groups with six mice per group were formed:

- group A: non injected, non irradiated
- group B: injected, non irradiated
- group C: non injected, irradiated: 3 x 2 Gy
- group D: non injected, irradiated: 1 x 5 Gy
- group E: injected, irradiated: 3 x 2 Gy
- group F: injected, irradiated: 1 x 5 Gy

In figure 2, relative tumor growth in each group is expressed as the median of the Vt:Vo ratio (where Vt is the median tumor volume on a given day during the treatment and Vo is the mean tumor volume at the beginning of the treatment). Tumor growth delays have been adjusted with exponential fit and errors bars are not shown for clarity.

The *in vivo* experiments clearly show that injection of DRIL 32 PEG (group B) has no effect on tumor progression *per se,* if compared to control mice (group A). By contrast, when the DRIL treatment is combined to fractionated irradiation (groups E and F), tumor growth is strongly delayed when compared to mice treated with irradiation alone (groups C and D), demonstrating synergistic antitumor effect of the combined treatment.

Moreover, the absence of apparent toxicity observed in mice, when injected locally with DRIL molecules, shows that this treatment can delay tumor growth or even arrest tumor progression, without damaging normal tissues or organs.

Example 4: Additional experiments

The experiments were repeated with the DRIL 32 PEG without Superfect and give similar results, indicating that the DRIL does not require Surpefect to act.

An assay of coupling DRIL treatment administered by feeding with a double chemotherapy (5FU and CPT 11) injected to K-ras -/Apc- mice together 3 times, 3 consecutive days spaced by a full week of rest, shows no toxic effect of the association (mouse kill/R-ras and Apx 163 8N).

In the absence of chemotherapy treatment, or after double chemotherapy alone the mice die at age of 6-7 months due to the development of spontaneous intestinal cancers. On the contrary, when the mice are fed by DRIL fragment compositions of the invention and submitted to the same double chemotherapy, no death is observed after 9 months.

**Claims**

1.  DNA repair induced lethality molecules molecules comprising a chemically modified backbone of at least 4-1000 bp, preferably 8-500 bp, and most preferably 12-100 bp.

2.  Molecules according to claim 1, having a phosphodiester backbone.

3.  Molecules according to claim 1 or 2, further comprising sugar mimetics such as cyclobutyls or other carbocyclics or hexitol in place of the pentofuranosyl group.

4.  Molecules according to any one of claims 1 to 3, further comprising one or several chemical groups at the end of each strand.

5.  Molecules according to claim 4, comprising one several phosphorothioates at the end of each strand.

6.  Molecules according to anyone of claims 1 to 5, wherein said backbone comprise methylphosphonates, phosphoramidates, morpholino nucleic acid, 2'-O,4'-C methylene/ethylene bridged locked nucleic acid, peptide nucleic acid (PNA), and short chain alkyl, or cycloalkyl intersugar linkages or short chain heteroatomic or heterocyclic intrasugar linkages of variable length.

7. Molecules according to any one of the preceding claims, wherein the fragments are based on natural nucleotides, either 2'-deoxynucleotides or 2'-ribonucleotides, and optionally comprise one or several modified nucleotides and/ or nucleobases other than adenine, cytosine, guanine, thymine and uracil.

8. Molecules according to claim 7, wherein said nucleobases are selected in the group comprising C5-methylcytosine, pseudoisocytosine, C5-propynyluracil, N7-deazaguanine, N7-glycosylated guanine, or alpha anomer.

9. Molecules according to any one of the preceding claims, further comprising at least one embedded element, which hampers DNA replication or repair process, said element(s) being incorporated in the center or at the end of the double-stranded molecules.

10. Molecules according to claim 9, comprising

- a) a polyethyleneglycol chain, preferably a hexaethyleneglycol chain, or any hydrocarbon chain, eventually interrupted and/or substituted by one or more heteroatoms e.g. oxygen, sulfur, nitrogen, or heterogroups comprising one or more of these heteroatoms;
- b) a unit which is a blocking element as it is not amenable by DNA polymerases or exonucleases, such as any 3'-modified nucleotides,
- c) a native oligonucleotide, such as $T_n$, when used in the loop of an hairpin fragment.

11. Adjuvant compositions to be used in association with a DNA breaking treatment, particularly radiotherapy or chemotherapy, said compositions comprising at least one DNA repair induced lethality molecule such as defined in any one of claims 1 to 10, in combination with a pharmaceutically acceptable carrier, in an efficient amount to be introduced in the tumor cell.

12. Adjuvant compositions according to claim 11, wherein said DNA repair induced lethality molecules are administered by any appropriate route such as oral, or intravenous, or intratumoral administration, or sub-cutaneous injections.

13. The use in association with a DNA breaking treatment, particularly radiotherapy or chemotherapy, of *native double stranded nucleic acid fragments* for making antitumoral drugs for treating tumors, particularly highly resistant tumors to radio-and/ or chemotherapies.

## Clonogenic cell survival after transfection and irradiation

Figure 1

Figure 2

**European Patent Office**

## PARTIAL EUROPEAN SEARCH REPORT

Application Number

which under Rule 45 of the European Patent Convention EP 03 29 2666
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | OMORI ET AL.: "suppression of a double-strand break repair gene, ku70, increases radio- and chemosensitivity in a human lung carcinoma cell line" DNA REPAIR 1, 2002, pages 299-310, XP002279444 * the whole document * --- | 1-13 | C12N15/11 |
| A | OHNISHI ET AL.: "In vitro and in vuvo potentiation of radiosensitivity of malignant gliomas by antisense inhibition of the RAD51 gene" BIOCHEMICAL AND BIOPHYSICAL RESEARCH COMMUNICATIONS, vol. 245, no. 2, 1998, pages 319-324, XP002279445 * page 320, left-hand column, paragraph 2 * --- -/-- | 1-13 | |

TECHNICAL FIELDS
SEARCHED (Int.Cl.7)

C12N

## INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 14 May 2004 | Hoff, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
................................................
& : member of the same patent family, corresponding
    document

EPO FORM 1503 03.82 (P04C07)

**European Patent**
**Office**

**PARTIAL EUROPEAN SEARCH REPORT**

Application Number

EP 03 29 2666

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | VERELLE AND BOURHIS: "Modulation de la réponse cellulaire aux radiations ionisantes: vers de nouvelles cibles moléculaires ?" CANCER/RADIOTHER, vol. 1, 1997, pages 484-493, XP002279446 * abstract * ----- | 1-13 | |
| | | | **TECHNICAL FIELDS SEARCHED** (Int.Cl.7) |

EPO FORM 1503 03.82 (P04C10)

| European Patent Office | INCOMPLETE SEARCH SHEET C | Application Number EP 03 29 2666 |
|---|---|---|

Although claim 13 are directed to a method of treatment of the human/animal body (Article 52(4) EPC), the search has been carried out and based on the alleged effects of the compound/composition.

--------------------

Claim(s) searched incompletely:
        1-13

Reason for the limitation of the search:

Present claims 1-13 relate to a product defined by reference to a desirable characteristic or property, namely  The abilit of the product to enhance the tumor sensitivity to direct or indirect DNA damaging anticancer therapies. The products defined in claim 1 are so called DNA repair induced lethality molecules.

The claims cover all productshaving this characteristic or property, whereas the application provides support within the meaning of Article 84 EPC and/or disclosure within the meaning of Article 83 EPC for only a one of such products (reference is made to example 1). In the present case, the claims so lack support, and the application so lacks disclosure, that a meaningful search over the whole of the claimed scope is impossible. Independent of the above reasoning, the claims also lack clarity (Article 84 EPC). An attempt is made to define the product by reference to a result to be achieved. Again, this lack of clarity in the present case is such as to render a  meaningful search over the whole of the claimed scope impossible. Consequently, the search has been carried out for those parts of the claims which appear to be clear, supported and disclosed, namely those parts relating to the the oligonucleotides of SEQ ID NOs:1 and 2.

SEQUENCE LISTING

```
<110>   INSTITUT CURIE

<120>   "Nucleic acids useful for triggering tumor cell lethalit
y"

<130>   CP/61061-1871

<140>   EP 03 292 666.9
<141>   2003-10-24

<160>   2

<170>   PatentIn version 3.1

<210>   1
<211>   32
<212>   DNA
<213>   Artificial

<400>   1
acgcacgggt gttgggtcgt ttgttcggat ct
        32


<210>   2
<211>   32
<212>   DNA
<213>   Artificial

<400>   2
tgcgtgccca caacccagca aacaagccta ga
        32
```